# EUROPEAN PATENT APPLICATION

(11) **EP 3 818 969 A1**
(43) Date of publication of application: **12.05.2021**
(21) Application number: 19207739.4
(22) Date of filing: 07.11.2019
(51) Int. Cl.: A61G 7/057, A61G 13/04, A61G 13/08, A61B 5/11, A61B 5/00

(54) **OPERATING TABLE, SYSTEM OF THE OPERATING TABLE AND AN EVALUATION UNIT, AND METHOD FOR OPERATING THE SYSTEM**

(71) Applicant: TRUMPF Medizin Systeme GmbH + Co. KG, 07318 Saalfeld (DE)
(72) Inventor: KÖHLER, Matthias, Batesville, IN 47006 (US); HARIDASAN, Shyamalakshmi, Batesville, IN 47006 (US); FALANGA, Lino, Batesville, IN 47006 (US)
(74) Representative: Prüfer & Partner mbB Patentanwälte · Rechtsanwälte

(57) **Abstract**

An operating table (2) comprises a tabletop (4) having a lying surface, and a plurality of sensors (10, 11) respectively configured to detect presence of a part of a patient's body on the tabletop (4) above the respective sensor (10, 11), wherein the sensors (10, 11) are configured to detect different physical characteristics of the part of the patient's body in order to determine the posture of the part of the patient's body or of the patient's body.

## Description

The invention relates to an operating table, a system of the operating table an evaluation unit, and a method for operating the system, in particular, to an operating table, a system of the operating table an evaluation unit, and a method for operating the system in connection with a determination of a patient's posture.

During surgical inventions, patients are usually under anesthesia and they are unconscious. Therefore, they are unable to express themselves about their condition and whether they are in pain due to their posture. However, in particular, upon long-term surgical interventions and pre-existing defects of body parts of the patient, there is the risk that decubitus occurs or is worsened.

In order to avoid decubitus or to reduce the ramifications in case of an existing decubitus or in case of an ulcer-prone body part, US 2019/0021650 suggests a pressure-sensing device containing a pressure-sensitive region.

However, also, joint pain or muscle pain can occur after the surgical invention if a limb is positioned in a manner which causes such pains.

Moreover, in particular, in case of extreme postures close to an edge of a tabletop of the operating table, there is a potential risk of falling from tabletop for the patient or the risk of tilting of the operating table.

Therefore, the object underlying the invention is to remedy the above disadvantages and to provide an operating table, a system of an operating table and an evaluation unit, and a method for operating the system enabling a safe surgical intervention without current or late complications for the patient.

The object is achieved by an operating table according to claim 1, a system according to claim 8, and a method according to claim 11. Advantageous further developments are included in the dependent claims.

According to an aspect of the invention, an operating table comprises a tabletop having a lying surface and a plurality of sensors respectively configured to detect presence of a part of a patient's body on the tabletop above the respective sensor, wherein the sensors are configured to detect different physical characteristics of the part of the patient's body.

By the detection of more than one physical characteristic of the part of the patient's body when detecting the presence of the part of the patient's body on the tabletop, a more detailed information about the posture of the patient is possible. Therefore, disadvantages caused by the posture can be determined in an early stage and, therefore, they can be avoided or diminished.

In an advantageous implementation of the operating table, the physical characteristics comprise a weight of at least a relevant part of the patient's body and a conductivity of the patient's body, and one kind of the sensors comprises a pressure sensor and another kind of the sensors comprises a capacitive proximity sensor.

By detecting the pressure onto the tabletop by means of the pressure sensor, the adverse consequences of an overstraining of an area of the skin of a body part can be avoided and, moreover, due to the detection of a body part which is not lying on the lying surface by the capacitive proximity sensor, also such body parts can be considered in the determination of posture of the entire body of the patient. Moreover, a change in distance of the body part can be detected and this information can be used for several determinations of the conditions of the posture during the surgical intervention.

In a further advantageous implementation of the operating table, the sensors are made at least partly, preferably completely, of a flexible material.

When being made of a flexible material, the sensors do not form rigid areas which, contrary to flexible areas, would deteriorate the contact conditions between the patient and the lying surface in order to avoid or diminish tissue damage.

In a further advantageous implementation of the operating table, the pressure sensor is made of a pressure-sensitive conductive sheet and/or the capacitive proximity sensor comprises a textile-based capacitive sensor.

The pressure sensor made of a pressure-sensitive conductive sheet and the capacitive proximity sensor comprising a textile-based capacitive sensor enable the provision of cost-effective sensors not deteriorating the contact conditions between the patient's body and the lying surface so that contact conditions to the patient's body are not worsened.

According to a further advantageous implementation of the operating table, it comprises at least one pad provided on the tabletop, and the sensors are integrated in the at least one pad.

By the integration of the sensors in the pad, the sensors are protected from mechanical damage and from adverse effects caused by disinfectants so that their life span can be increased.

In a further advantageous implementation, the operating table comprises several pads, and the sensors are integrated in more than one of the several pads.

In particular, when being provided with several tabletop sections being movable with respect to one another, the provision of several pads for the individual tabletop sections is advantageous. The sensors are then integrated in the pads which are crucial for determination of the requested information about the posture of the patient. In a further advantageous implementation, the sensors are arranged at predefined locations in a sensor array.

By arranging the sensors at predefined locations in a sensor array, the specific characteristic of the parts of the patient's body at the predefined locations can be detected to enable an evaluation of the characteristics of the parts of the patient's body at the predefined locations.

According to another aspect of the invention, a system of the operating table and an evaluation unit is provided, wherein the sensors are configured to respectively provide a presence signal when the presence of the part of the patient's body is detected by the respective sensor, and the evaluation unit is configured to evaluate the presence signal of the sensors at the predefined locations.

This system enables a determination whether a patient is anyway present and a determination where and in which orientation the patient is lying on the tabletop within the sensor array.

In an advantageous implementation of the system, the evaluation unit is configured to determine a posture of the part of the patient's body and/or of the patient's body from the predefined locations of the sensors providing the presence signal.

Due to this system, the posture of the parts of the patient's body located in the sensor array can be determined since values of physical characteristics at the predefined locations can be assigned to a specific part of the patient's body and the behavior of the body part can be determined.

In a further advantageous implementation of the system, the evaluation unit is configured to determine a pressure exerted by the part of the patient's body at the predefined locations.

When the pressure at the predefined locations is determined, a risk for decubitus can be determined, on the one hand, by the amount of the pressure, and, on the other hand, by an allocation of a location of the pressure with a location and posture of the part of the patient's body so that it is possible to verify if the location of the pressure is a place on the body part being endangered for decubitus.

According to a further aspect of the invention, a method for operating the system includes the step: determining the posture of the patient's body by the evaluation unit from the presence signal of the sensors at the predefined locations.

The posture of the patient's body located in the sensor array is determined in order to determine the presence or absence of the patient's body and the posture for the ability to record or to immediately use the information about the posture, e.g., for providing warning signals.

In an advantageous implementation of the method, it includes the step: signaling a potential risk of falling from the tabletop for the patient from the posture of the patient's body.

By this step, the patient's safety can be increased since a movement of the patient, e.g., when the posture of the patient is changed or when he shifts his body on the tabletop, can be determined and, when exceeding certain thresholds, this movement can be signaled to be dangerous.

In a further advantageous implementation of the method, it includes the step: determining a center of gravity of the patient's body by the evaluation unit; and signaling a potential risk for tilting of the operating table.

Due to this step, the safety of the patient and, also, of the surgery personnel can be increased since a potential risk for tilting of the operating table can be signaled when a certain threshold concerning the location of the center of gravity of the patient is exceeded.

In another advantageous implementation of the method, a time-dependent posture of the patient's body is stored.

When storing the time-dependent posture of the patient's body, this data can be used for comprehending the postures of the patient in the surgical procedure for predicting potential defects of body parts and joint pain or muscle pain of the patient and for initiating prophylactic measures.

In a further advantageous implementation of the method, it includes the steps: determining potential decubitus risk from the time-dependent posture of the patient's body and pressure values of a pressure sensor; and signaling the potential decubitus risk.

By executing these steps, the potential decubitus risk can be determined and signaled and, therefore, an immediate change of the posture of the patient's body or a subsequent prophylactic measure can be performed in order to avoid or decrease such problems for the patient.

Subsequently, the invention is elucidated by means of embodiments referring to the drawings.

In particular,
- Fig. 1: shows a system according to the invention comprising an operating table and an evaluation unit; and
- Fig. 2: shows a flowchart of a method for operating the system.

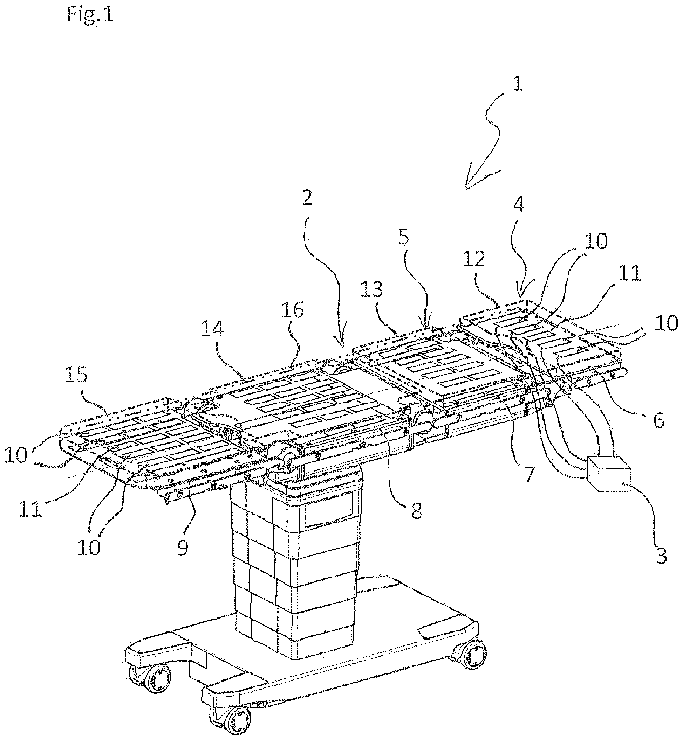

- **Fig. 1**: shows a system 1 comprising an operating table 2 and an evaluation unit 3.

The operating table 2 comprises a tabletop 4 having a lying surface 5. The tabletop 4 comprises four tabletop sections 6, 7, 8, 9. In particular, the operating table 2 comprises a head plate 6, a back plate 7, a seat plate 8 and a leg plate 9. The tabletop sections 6, 7, 8, 9 are movable with respect to one another.

Further, the operating table 2 comprises a plurality of sensors 10, 11. The sensors 10 and the sensors 11 are different kinds of sensors and detect respectively different physical characteristics of a part of a patient's body. In Fig. 1, the quantity of the respective sensors 10, 11 and the location of the sensors 10, 11 are merely depicted in principle, wherein, for reasons of clarity and comprehensibility, not all of the depicted sensors 10, 11 are denoted with a reference sign. In fact, the quantity and the location of the sensors 10, 11 is determined such that, by the sensors 10, 11, presence of a part of a patient's body on the tabletop 4 above the respective sensor 10, 11 is detected. In alternative embodiments, the quantity and the locations of the sensors 10, 11 are different from the depicted illustration.

The physical characteristics comprise a weight of the part of the patient' body and a conductivity of the patient's body. One kind of the sensors 10 comprises a pressure sensor and another kind of the sensors 11 comprises a capacitive proximity sensor. In alternative embodiments, other sensors than the capacitive proximity sensors and the pressure sensors are used, e.g., a camera instead of the capacitive proximity sensor.

The sensors 10, 11 are made of a flexible material. In particular, the pressure sensor is made of a pressure-sensitive conductive sheet and the capacitive proximity sensor comprises a textile-based capacitive sensor. In alternative embodiments, the sensors are not made of a flexible material or do not comprise a flexible material so that they are made of or comprise a rigid material. Further alternatively, other pressure-sensitive materials or other materials providing the function of a capacitive proximity sensor are used.

The operating table 2 comprises several pads 12, 13, 14, 15 each being provided on one of the tabletop sections 6, 7, 8, 9. The pads 12, 13, 14, 15 form the lying surface 5 of the tabletop 4. The sensors 10, 11 are integrated in the pads 12, 13, 14, 15 of the head plate 12, the back plate 13, the seat plate 14, and the leg plate 15. In alternative embodiments, only one pad is provided for one or several of the tabletop sections 6, 7, 8, 9. Hence, the sensors 10, 11 are merely integrated in one pad. Further alternatively, the sensors 10, 11 are integrated in more than one but in not all of the pads 12, 13, 14, 15 or they are not integrated but are provided on or below the pads 12, 13, 14, 15. The sensors 10, 11 are arranged at predefined locations in a sensor array 16. In this embodiment, the sensor array 16 covers the entire surface of the pads 12, 13, 14, 15 of the head plate 6, back plate 7, the seat plate 8, and the leg plate 9. In alternative embodiments, the sensor array 16 merely covers portions of the respective pads 12, 13, 14, 15 or it does not cover all of the pads 12, 13, 14, 15.

The sensors 10, 11 are connected to the evaluation unit 3. For reasons of clarity and comprehensibility, only the sensors 10, 11 of the head plate 6 are depicted as being connected to the evaluation unit 3. Actually, all of the sensors 10, 11, are connected to the evaluation unit 3. The evaluation unit 3 evaluates the presence signals of the sensors 10, 11 at the predefined locations. In particular, the evaluation unit 3 determines a posture of the part of the patient's body from the predefined locations of the sensors 10, 11 providing the presence signal. Alternatively, the evaluation unit 3 determines a posture of the entire patient's body from the predefined locations of the sensors 10, 11 providing the presence signal. Furthermore, it is possible to differentiate between the patient's body and any item lying on the tabletop 4.

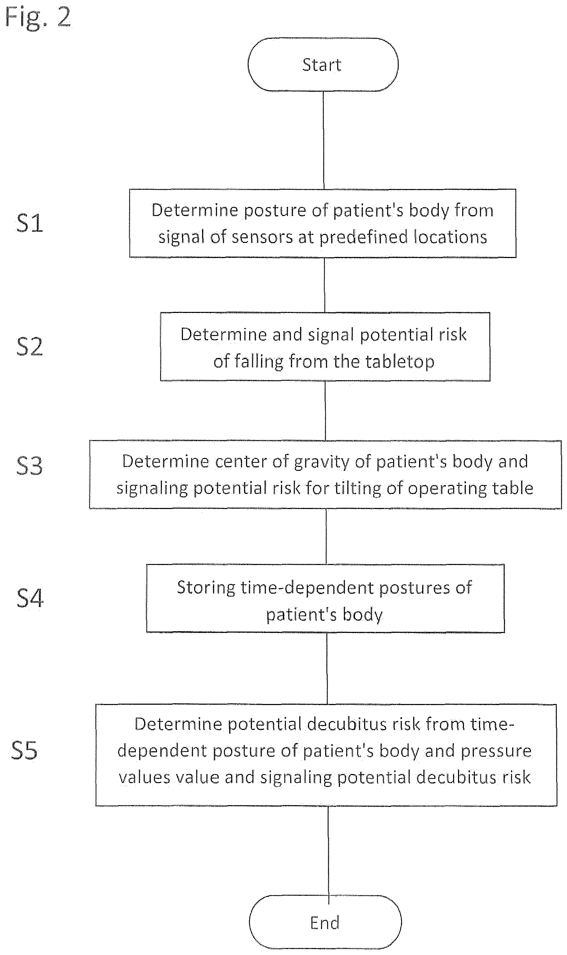

**Fig. 2** shows a flowchart of a method for operating the system. In use, the evaluation unit 3 determines the posture of the patient's body by the evaluation unit from the presence signals of the sensors at the predefined locations.

In step S1, the posture of the patient's body is determined from the posture of the body parts of the patient's body. The posture of the body parts of the patient's body is respectively determined by identifying the body part due to its shape and location detected by the sensors 10, 11 at the predefined locations with respect to the shape and location of other body parts detected by the sensors 10, 11.

In an optional step S2, by the determination of the location and a motion of the several body parts, i.e. the posture of the patient's body, by the sensors 10, 11, a potential risk of falling from the tabletop for the patient can be determined from the posture of the patient's body by the evaluation unit 3 and be signaled. This signaling is, e.g., performed by an audible signal.

In a further optional step S3, based on the signals of the pressure sensors, a center of gravity of the patient's body can be determined by the evaluation unit 3. In case that the location of the center of gravity of the patient's body exerts a location threshold, a potential risk for tilting of the operating table is signaled, e.g., by an audible signal.

Furthermore, in an optional step S4, a data of time-dependent postures of the patient's body is stored. These stored data can be used for documenting the patient's posture during a surgical intervention. Hence, e.g., a prospective intervention after the surgical intervention for avoiding or diminishing decubitus can be performed. Also, optionally, other interventions concerning circulatory or for avoiding joint pain can be performed.

From the results of the time-dependent posture of the patient's body and the pressure values of the pressure sensors, in an optional step S5, a potential decubitus risk can be determined during the surgical intervention. The risk can be signaled by, e.g., an audible signal so that the posture of the patient can be changed or other measures, e.g., providing a specific cushion, can be performed. Furthermore, it is possible to give other systems access to that data.

While the invention has been illustrated and described in detail in the drawing and the foregoing description, such illustration and description are to be considered illustrative or exemplary and not restrictive. The invention is not limited to the disclosed embodiments. From reading the present disclosure, other modifications will be apparent to a person skilled in the art. Such modifications may involve other features, which are already known in the art and may be used instead of or in addition to features already described herein. In the claims, the word "comprising" does not exclude other elements or steps, and the indefinite article "a" or "an" does not exclude a plurality.

## Claims

1. An operating table (2) comprising
a tabletop (4) having a lying surface, and
a plurality of sensors (10, 11) configured to detect presence of a part of a patient's body on the tabletop (4) above the respective sensor (10, 11),
wherein
the sensors (10, 11) are configured to detect different physical characteristics of the part of the patient's body.

2. The operating table (2) of claim 1, wherein
the physical characteristics comprise a weight of at least a relevant part of the patient's body and a conductivity of the patient's body, and
one kind of the sensors (10) comprises a pressure sensor and another kind of the sensors (11) comprises a capacitive proximity sensor.

3. The operating table (2) of claim 2, wherein
the sensors (10, 11) are made at least partly, preferably completely, of a flexible material.

4. The operating table (2) of claim 3, wherein
the pressure sensor is made of a pressure-sensitive conductive sheet and/or the capacitive proximity sensor comprises a textile-based capacitive sensor.

5. The operating table (2) of anyone of the preceding claims, wherein
the operating table (2) comprises at least one pad (12, 13, 14, 15) provided on the tabletop(4) , and
the sensors (10, 11) are integrated in the at least one pad (12, 13, 14, 15).

6. The operating table (2) of claim 5, wherein
the operating table (2) comprises several pads (12, 13, 14, 15), and
the sensors (10, 11) are integrated in more than one of the several pads (12, 13, 14, 15).

7. The operating table (2) of anyone of the preceding claims, wherein
the sensors (10, 11) are arranged at predefined locations in a sensor array (16).

8. A system (1) of the operating table (2) of claim 7 and an evaluation unit (3), wherein
the sensors (10, 11) are configured to respectively provide a presence signal when the presence of the part of the patient's body is detected by the respective sensor (10, 11), and
the evaluation unit (3) is configured to evaluate the presence signal of the sensors (10, 11) at the predefined locations.

9. The system of claim 8, wherein
the evaluation unit is configured to determine a posture of the part of the patient's body and/or of the patient's body from the predefined locations of the sensors providing the presence signal.

10. The system (1) of claim 9 comprising an operating table (2) according to claim 2, wherein
the evaluation unit (3) is configured to determine a pressure exerted by the part of the patient's body at the predefined locations.

11. A method for operating the system (1) of claim 9 or 10, the method including the step:
determining the posture of the patient's body by the evaluation unit (3) from the presence signals of the sensors (10, 11) at the predefined locations.

12. The method of claim 11 further including the step:
determining and signaling a potential risk of falling from the tabletop (4) for the patient from the posture of the patient's body.

13. The method of claim 12 further including the steps:
determining a center of gravity of the patient's body by the evaluation unit (3); and
signaling a potential risk for tilting of the operating table (2).

14. The method of anyone of claims 11 to 13 further including the step:
storing a time-dependent posture of the patient's body.

15. The method of claim 14 further including the steps:
determining potential decubitus risk from the time-dependent posture of the patient's body and pressure values of a pressure sensor; and
signaling the potential decubitus risk.
